# EUROPEAN PATENT SPECIFICATION

(11) **EP 2 198 958 B1**
(45) Date of publication and mention of the grant of the patent: **08.11.2017**
(21) Application number: 08172023.7
(22) Date of filing: 17.12.2008
(51) Int. Cl.: B01J 23/72, B01J 23/78, B01J 23/83, B01J 35/10, C07C 17/156, C07C 19/045, B01J 8/02, B01J 27/138, B01J 35/02

(54) **Catalysts for oxychlorination of ethylene to 1.2-dichloroethane**
Katalysatoren zur Oxychlorierung von Ethylen zu 1,2-Dichlorethan
Catalyseurs pour l'oxychloration de l'éthylène en 1,2-dichloroéthane

(43) Date of publication of application: 23.06.2010
(73) Proprietor: Clariant Prodotti (Italia) SpA, 20124 Milano (IT)
(72) Inventor: Orsenigo, Carlo, 20155 Milano (IT); Casagrande, Francesco, 28100 Novara (IT); Civati, Marco, 28045 Invorio (NO) (IT)
(74) Representative: Modiano, Micaela Nadia

(56) References cited:
- EP-A- 1 645 332
- WO-A-03/066214
- WO-A-2008/054564
- US-A- 4 414 136
- US-A- 5 166 120

## Description

The present invention relates to catalysts usable in fixed-bed oxychlorination of ethylene to 1.2-dichloroethane (DCE) in form of granules having hollow geometrical configuration endowed with a particular pore volume distribution and to the hollow carriers used for said catalysts.

The oxychlorination of ethylene to DCE is carried out, as it is known, either in fluid bed or in fixed bed. In the first case, more uniform distribution of the temperatures in the reactor is obtained, in the other case, the management of reaction parameters is easier but, due to the low exchange coefficient among the catalyst granules and between the granules and the reaction gas, localized hot spot temperatures can occur having detrimental effects on the selectivity and useful life of the catalyst.

Hollow cylindrical granules are normally used which, thanks to S/V ratio (geometric surface to volume ratio) higher than that of the spheres and solid cylinders allow to obtain more efficient heat exchange and lower pressure drop through the catalytic bed, and consequently better temperature control along the bed and increased productivity of industrial reactors.

In spite of the above advantages, a hollow cylindrical granule has to be designed carefully since, otherwise several disadvantages become evident.

For example, if the ratio of the external to internal diameter (De/Di) of the hollow cylinder is greater than a certain value, the granules become too fragile and the bulk density of the catalyst decreases resulting in a decreased conversion per unit volume of the catalytic bed due to the lower presence of total content of the active catalyst phase.

A too high increase of De or the length of the cylinders maintaining constant the De/Di ratio can cause an inhomogeneous loading of the catalyst inside the tubes of the reactor and possible breakage of the granules with consequent increase of the pressure drop.

A catalyst in form of cylinders having De from 4 to 7 mm, Di from 2.0 to 2.8 nm, height from 6.1 to 6.9 mm is described in EP 1 053 789 A1.

This catalyst is reported to be advantageous with respect to the catalysts in form of cylinders having length shorter than the external diameter described in US P 4740644 and the catalysts having longer length than the external diameter (US P 5166120).

The catalysts of the latter cited US patent are disclosed to be useful for oxychlorination of ethylene and contain copper and alkali ions on an annular carrier and are also characterized by a total pore volume of 0.6 to 1.0 ml/g, wherein no pores smaller than 4 nm are present and at least 80% of the pore volume is formed of pores with a diameter of 8 to 20 nm, the remainder being pores with diameter of more than 20 nm and up to 200 nm.

The catalyst of US P 5166120, according to the consideration made in EP 1 053 789, has the disadvantage of a too high bed void fraction which implies a lower amount of catalytic material present in the bed and consequently a lower specific productivity to DCE (g DCE/g catalyst.h) combined with high pressure drop due to breakage of the catalyst granules during the loading step.

US-A-4 414 136 discloses oxychlorination catalysts in form of solid granules, especially spheres, presenting bimodal peak pore distribution one peak corresponding to diameters of 15-25 nm, the other to 1000-12000 nm having no effect on the catalyst activity.

### Objects

It is an object of the present invention to provide catalysts for the oxychlorination of ethylene to DCE performed in fixed bed, in form of hollow granules comprising copper chloride and chlorides of the metals selected from the alkali metals, the alkaline earth metals and the rare earth metals supported on gamma alumina hollow cylindrical granules, endowed with satisfactory performance in terms of selectivity and conversion and capable of providing specific productivity to DCE (g DCE/g catalyst.h) higher than that of catalysts having the same geometrical parameters (shape and size), and composition.

Other objects will be apparent from the following description of the invention.

### Description of the invention

The catalysts of the present invention, which are in form of hollow granules having definite geometrical configuration and comprise copper chloride and at least one or more chlorides of the metals selected from the group of the alkali metals, alkaline earth metals and rare earth metals supported on alumina hollow cylindrical granules, are characterized by a macropore volume fraction of the granules of at least 20% of the total pore volume and up to 40%, wherein the diameter of the pores at the maximum of the macropore volume distribution curve is from 800 nm up to 1500 nm. The bulk density preferably is comprised from 0.80 g/ml to 0.65 g/ml, more preferably from 0.7 g/ml to less than 0.78 g/ml.

The boehmite used for preparing the catalysts of the invention has d₅₀ of at least 100 µm and up to 170 µm and a fraction of particles of less than 100 µm lower than 50 wt%.

An example of usable boehmite is the commercial boehmite Pural SCC 150 manufactured by SASOL AG - Germany.

This boehmite has particle size distribution (wt%) evaluated by ponderal sieve screening as follows:
> 250 µm 0.7%
250-100 µm 56.3%
100-63 µm 27.3
<63 µm 15.7%

The d₅₀ value (average) is 130 µm, the d₉₀ (average) 209 µm. The volume of particles having diameter less than 100 µm is 30%; the volume of particles with diameter less than 250 µm is 98%.

The compression-shaped hollow cylindrical granules obtained from the above boehmite, calcined at 650°C, 700°C and 800°C to obtain conversion to gamma alluminia, have the following characteristics:
- surface area (BET) of 239, 208 and 183 m²/g calcined at the above mentioned temperatures, respectively;
- pore volume (BET) 0.41 - 0.43 ml/g;
- total pore volume = 0.48 - 0.52 ml/g (calculated as difference between the reciprocals of the apparent density (DA) and the real density (RD) = 1/AD - 1/RD);
- macropore volume = 0.1 ml/g (Hg porosimetry);
- pore diameter (MPS) at the maximum of the macropore volume distribution curve = 1068 nm.

Diameters up to 1600 nm can be obtained using boehmite having d₅₀ higher than 150 µm. The catalysts obtained from this boehmite are endowed with satisfactory good performance and sufficient mechanical properties (axial and radial crush resistance).

The boehmite usable in preparing the catalysts of the invention is obtainable according to know methods, by dissolution of aluminum in hexanol via a modified Ziegler alcohol process: the obtained boehmite slurry is then dried by spray drying.

The characteristics of the hollow cylindrical gamma alumina granules prepared from boehmite Pural SCC 150 hollow granules, having external diameter (De) about 5 mm, internal diameter (De) 2.5 mm, height about 5 mm, as reported in Table 1, wherein the characteristics are also reported of gamma alumina granules having the same size and shape as the granules of gamma alumina from boehmite Pural SCC 150, obtained from SASOL Pural SB1 boehmite having d₅₀ (average) 43 µm and d₉₀ = (average) 119 µm, volume of the particles having diameter less than 100 µm of 83.9% and having diameter less than 250 µm = 100%.

**Table 1**

| Properties | | Alumina carrier from Pural SCC 150 | Alumina carrier from Pural SB 1 |
|---|---|---|---|
| Temp. Calcn | °C | 700 | 600 |
| S.A. | m²/g | 208 | 207 |
| Pore Vol.(BET) | ml/g | 0.41 | 0.46 |
| Total Pore Vol. | ml/g | 0.51 | 0.50 |
| Macro Pore Vol. | ml/g | 0.1 | 0.06 |
| Macro Pore Vol % on total | | 20 | 12 |
| Diameter MPS | nm | 1068 | 401 |
| Crush Res. axial | Kg/prt | 86 ±18 | 73 ± 11 |
| Crush Res. radial | Kg/prt | 1.9 ± 0.4 | 2.0 ± 0.4 |

The granules are prepared by compression-shaping mixtures of powder boehmite with a lubricant such as for example aluminum stearate in amount of 3-6 wt%.

The obtained shaped granules are calcinated at temperature from about 600° to 800°C to convert the boehmite into gamma alumina, which are then impregnated with an aqueous solution of the metal chlorides-catalyst components. The impregnation is preferably carried out using a volume of solution equal or lower than the total pore volume of the alumina granules.

The amount of the chlorides present in the catalyst expressed as metal is 3 - 12 wt% Cu, 1 - 4 wt% alkali metal, 0.05 - 2wt% alkaline earth metal, 0.1 - 3 wt% rare earth metal.

Preferably the amount of Cu is from 4 to 10 wt%, and the alkali metal is potassium and/or cesium used in amount of 0.5 to 3 wt%, the alkaline earth metal is magnesium in amount of 0.05 to 2 wt% and the rare earth metal is cerium in amount of 0.5 to 3 wt%.

The hollow granules comprise at least one through bore parallel to the axis of the granule. In the case of cylindrical granules, the De diameter is from 4 to 6 mm, the Di diameter of 1 to 3 mm and the height of 4 to 7 mm.

Pellets having trilobed cross-section with the lobes provided with through bores parallel to the axis of the granules are conveniently used.

Representative properties of the catalyst granules of the invention are reported in Table 2, while in Table 3 the results are reported of the catalysis tests obtained with the catalysts of the examples.

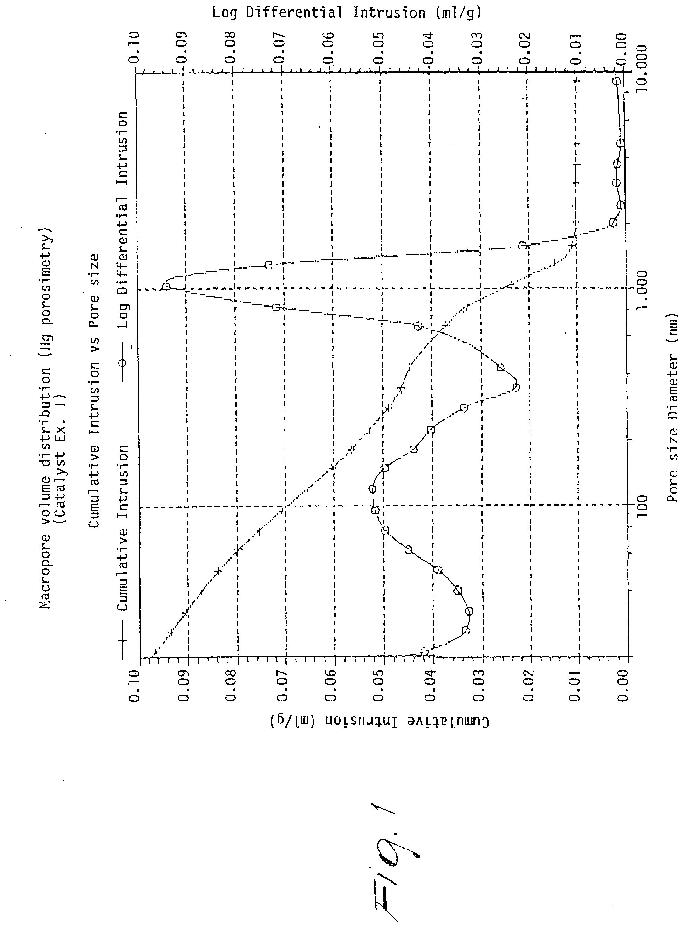

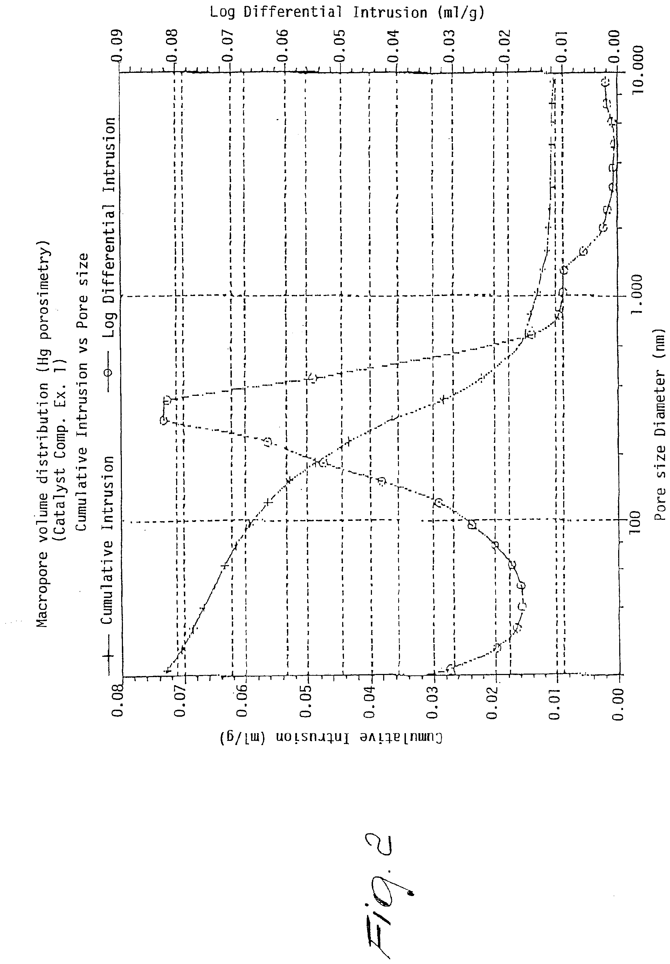

In Figures 1 and 2, are reported the macropore volume distributions (Hg porosimetry) of a catalyst according to the invention (Fig. 1, cat. Ex. 1) and a prior art catalyst (Fig. 2, cat. comp. Ex. 1). In the ordinate axes, is reported the cumulative pore volume, (left side) while, in the right side ordinates, the pore volume increase (logarithmic) vs. the variation of the pore diameter is reported (log. d PV/d PD where PV is the pore volume, PD the pore diameter).

The oxychlorination of ethylene to DCE using the catalysts granules of the invention is carried out in fixed bed according to known methods using air or oxygen as oxidizer, at temperatures from 200°C to 300°C, using overall feed molar ratios C₂H₄/HCl/O₂ of 1 : 1.99 : 0.51 when using air and of 1 : 0.71 : 0.18 when using oxygen.
Preferably, the molar ratios HCl/C₂H₄, O₂/C₂R₄ and HCl/O₂ are respectively 0.15 to 0.50, 0.04 to 0.1 and 3.20 to 5.8 when using oxygen and the process is carried out in three reactors in series, wherein the third reactor is loaded with a fixed bed formed or comprising the catalyst granules according to the invention.

### Measurements

The macropore volume is measured by Hg-porosimetry: the micro and the meso pores by BET nitrogen adsorption - desorption.

The bulk density (called also apparent packing density) is measured according to ASTM method D 4164-82.

The following examples are given to illustrate but not to limit the scope of the invention.

### Example 1

400 g of alumina hollow cylinders with De = 5 mm, Di = 2.5mm and height = 5 mm obtained by calcination at 700 °C of cylinders prepared by compression-shaping of a powder of boehmite Pural SCC 150 mixed with 6 wt% of aluminum tristearate, having S.A. (BET) of 208 m²/g, total pore volume of 0.51 ml/g, are impregnated in rotating jar of 5 1 at room temperature up to 50°C, with 200 ml of an aqueous solution containing
CuCl₂*2H₂O = 110.8 g
KCl = 7.9 g
MgCl₂*6H₂O = 2.1 g
HCl 37 wt% = 8.0 ml

Remaining = demineralized water up to 200 ml.

The impregnated granules were dried in an oven with the following cycle = 1h at 60°C, 2hs at 80°C, 3hs at 100°C and 16hs at 150° C.

The characteristics of the granules are reported in Table 2 wherein the characteristics of the catalysts of comparative Example 1 and 2 are also reported.

The results of the catalysis test are reported in Table 3.

### Comparative Example 1

300 g alumina hollow cylinders having the same size and shape as the cylinders of Example 1, obtained by calcination of crude cylindrical granules prepared by compression-shaping of boehmite Pural SB1 mixed with 4 wt% of aluminum tristearate were impregnated in rotating jar of 5 1 at r.t. with 150 ml of an aqueous solution containing :
CuCl₂*2H₂O = 83.1g
KCl = 5.9 g
MgCl₂*6H₂O = 1.5 g
HCl 37 wt% = 6.0 ml
Remaining = demineralized water up to 150 ml.

The impregnated granules are dried as described in Example 1.

The results of the catalysis test carried out under the same conditions as of Example 1 are reported in Table 3.

### Comparative Example 2

Two commercial catalysts having similar size and shape as the catalyst of Example 1 and similar composition were used under the same catalysis test conditions as in Example 1.

The results of the test are reported in table 3.

**Table 2**

| Catalyst Properties | | Catalyst from Pural SCC 150 | Catalyst from Pural SB1 | Com. Catalyst 1 | Com. Catalyst 2 |
|---|---|---|---|---|---|
| Cu | wt% | 7.98 | 7.95 | 7.92 | 7.68 |
| Mg | wt% | 0.05 | 0.05 | 0.22 | 0.12 |
| K | wt% | 0.78 | 0.8 | 0.86 | 0.84 |
| S.A. (BET) | m²/g | 115 | 129 | 135 | 102 |
| Pore Vol. (BET) | ml/g | 0.27 | 0.29 | 0.30 | 0.21 |
| Particle Density | g/ml | 1.55 | 1.55 | 1.47 | 1.78 |
| True Density | g/ml | 3.19 | 3.21 | 3.23 | 3.14 |
| Total Pore Vol. | ml/g | 0.33 | 0.33 | 0.37 | 0.24 |
| Macro Pore Vol. | ml/g | 0.07 | 0.06 | 0.07 | 0.05 |
| % Macro P. Vol% on total Vol. | | 21 | 18 | 19 | 20 |
| Diameter M.P.S. (*) | | 1050 | 310 | 660 | 480 |
| Weight (100 prt) | | 10.42 | 10.60 | 9.60 | 13.12 |
| Diameter | mm | 4.89 | 4.91 | 4.69 | 5.01 |
| Height | mm | 4.74 | 4.81 | 4.67 | 4.95 |
| Apparent Bulk density g/ml | | 0.72 | 0.78 | 0.78 | 0.86 |
| Bed void fraction (**) | | 0.54 | 0.50 | 0.47 | 0.52 |

| | | | | | |
|---|---|---|---|---|---|
| (*) M.P.S. = diameter of the pores of the maximum value of the macropore volume distribution Macropores = pores having diameter of more than 50 nm up to 10.000 nm. (**) Bed void fraction = 1 - Apparent Bulk Density/Particle density. | | | | | |

**Table 3**

| Catalyst Performance | | | | | | |
|---|---|---|---|---|---|---|
| Catalysts | | | Ex 1 | Comp. Ex. 1 | Com. Cat. 1 | Com Cat. 2 |
| Cat. bed | Height | cm | 80 | 80 | 80 | 80 |
| | Volume | ml | 420 | 420 | 420 | 420 |
| Cat. loading | | g | 303.9 | 327.5 | 360.6 | 325.6 |
| Cat. Bulk density | | g/ml | 0.72 | 0.78 | 0.86 | 0.78 |
| | | | | | | |
| Feed | Tot. feed | Nl/h (*) | 761.3 | 756.7 | 772.9 | 765.9 |
| | HCl | Nl/h | 70.3 | 69.8 | 73.5 | 72.9 |
| Feed molar ratio | HCl/C₂H₄ | | 0.30 | 0.30 | 0.31 | 0.31 |
| | O₂/C₂H₄ | | 0.09 | 0.09 | 0.09 | 0.09 |
| Termal profile cat. bed | T10 cm | °C | 269 | 269 | 304 | 256 |
| | T20 cm | °C | 287 | 291 | 317 | 279 |
| | T30 cm | °C | 272 | 275 | 283 | 268 |
| | T70 cm | °C | 220 | 223 | 218 | 228 |
| Conversion | HCl | %mol | 99.0 | 98.7 | 96.4 | 95.0 |
| | C₂H₄ | %mol | 15.3 | 15.6 | 15.6 | 15.2 |
| | O₂ %mol | | 96.5 | 95.6 | 97.9 | 92.5 |
| Purity | EDC %mol | | 99.58 | 99.53 | 99.57 | 99.51 |
| C₂H₄ mol. select. | CO % | | 1.18 | 1.40 | 1.97 | 1.21 |
| | CO₂% | | 1.46 | 1.88 | 1.68 | 1.40 |
| | Eth. Chl | | 0.04 | 0.04 | 0.07 | 0.05 |
| | EDC % | | 96.96 | 96.27 | 95.93 | 96.92 |
| Specific Productivity C₂H₄ to EDC refer cat. bed. Vol. | | gEDC/ml cat.h | 0.37 | 0.36 | 0.37 | 0.36 |
| Specific Productivity refer cat. weight | | gEDC/g cat.h | 0.51 | 0.46 | 0.43 | 0.47 |

| | | | | | | |
|---|---|---|---|---|---|---|
| (*) Total feed: C₂H₄= 30.4 vol.%, O₂= 2.7 vol.% HCl = 9.5 vol.%, N₂ = 57.4 vol.%. | | | | | | |

## Claims

1. Catalysts for the oxychlorination of ethylene to 1.2-dichloroethane in form of hollow granules having geometrical configuration and comprising copper chloride and at least one or more chlorides of metals selected from the group consisting of the alkali metals, alkaline earth metals and rare earth metals in amount expressed as metal , of 3-12 wt% copper, and for the other chlorides when present alone or in mixture of 1-4 wt% of alkali metals, 0.05-2 wt% alkaline earth metals and 0.1 - 3 wt% rare earth metals, said chlorides being supported on alumina granules, **characterized in that** the pore volume fraction of the macropores having diameter higher than 50 nm and up to 10,000 nm of the catalyst granules is from 20% to 40% of the total pore volume and the diameter of the pores at the maximum of the macropore volume distribution curve is at least 800 nm and up to 1500 nm, wherein the macropore volume is measured by Hg porosimetry.

2. Catalysts according to claim 1 having bulk density from 0.65 to 0.8 g/ml.

3. Catalyst according to any one of claims 1 and 2, wherein the content of copper is from 4 to 10 wt% and wherein the alkali metal is potassium and/or cesium, the alkaline earth metal is magnesium, and the rare earth metal is cerium.

4. Catalyst according to any one of claims 1 to 3 , wherein the amount of potassium and/or cesium is 0.5-3 wt%, the amount of magnesium is 0.05-, 2 wt% and of cerium is 0.5-3 wt%.

5. Catalyst according to any one of claims 1 to 4, having cylindrical hollow form with external diameter of 4-6 mm, internal diameter 1-3 mm and height 4 -7 mm.

6. Catalyst according to any one of claims 1 to 4 having trilobed cylindrical cross-section provided of lobes each of which with a through bore parallel to the axis of the granule and equidistant from the axes of the other lobes.

7. Use of catalyst granules according to claims 1 to 6 for the oxychlorination of ethylene to 1,2 dichloroethylene.

## Patentansprüche

1. Katalysatoren für die oxychlorierung von Ethylen zu 1.2-Dichlorethan in Form von hohlen Körnchen mit geometrischer Gestalt und umfassend Kupferchlorid und mindestens eines oder mehrere Chloride von Metallen gewählt aus der Gruppe bestehend aus den Alkalimetallen, Erdalkalimetallen und Seltenerdmetallen in der Menge ausgedrückt als Metall, von 3-12 Gewichts% Kupfer, und für die anderen Chloride, wenn sie alleine oder in Mischung vorhanden sind, von 1-4 Gewichts% von Alkalimetallen, 0,05-2 Gewichts% Erdalkalimetallen und 0,1-3 Gewichts% Seltenerdmetallen, wobei die Chloride auf Aluminiumoxidkörnchen aufgelagert sind, **dadurch gekennzeichnet, dass** der Porenvolumenanteil der Makroporen mit einem Durchmesser von größer als 50 nm und bis zu 10,000 nm der Katalysatorkörnchen von 20% bis 40% des Gesamtporenvolumens ausmacht, und der Durchmesser der Poren am Maximum der Makroporenvolumenverteilungskurve mindestens 800 nm und bis zu 1500 nm ist, worin das Makroporenvolumen durch Hg Porositätenmessung gemessen wird.

2. Katalysatoren gemäß Anspruch 1 mit einer Bulkdichte von 0,65 bis 0,8 g/ml.

3. Katalysator gemäß irgendeinem der Ansprüche 1 und 2, worin der Gehalt an Kupfer von 4 bis 10 Gewichts% ist, und worin das Alkalimetall Kalium und/oder Cäsium ist, das Erdalkalimetall Magnesium ist und das Seltenerdmetall Cer ist.

4. Katalysator gemäß irgendeinem der Ansprüche 1 bis 3, worin die Menge an Kalium und/oder Cäsium 0,5-3 Gewichts% ist, die Menge an Magnesium 0,05-2 Gewichts% ist und an Cer 0,5-3 Gewichts% ist.

5. Katalysator gemäß irgendeinem der Ansprüche 1 bis 4, mit zylindrischer hohler Form mit äußerem Durchmesser von 4-6 mm, innerem Durchmesser 1-3 mm und Höhe 4-7 mm.

6. Katalysator gemäß irgendeinem der Ansprüche 1 bis 4 mit dreilappigem zylindrischem Querschnitt ausgestattet mit Lappen, von denen jeder eine Durchbohrungsparallele zu der Achse des Körnchens hat und gleich weit entfernt ist von den Achsen der anderen Lappen.

7. Verwendung von Katalysatorkörnchen gemäß Ansprüchen 1 bis 6 für die Oxychlorierung von Ethylen zu 1,2-Dichlorethylen.

## Revendications

1. Catalyseurs d'oxychloration d'éthylène en 1,2-dichloro-éthane, sous forme de granules creux dotés d'une certaine configuration géométrique et comprenant un chlorure de cuivre et au moins un ou plusieurs chlorures de métaux choisis dans l'ensemble formé par les métaux alcalins, les métaux alcalino-terreux et les métaux des terres rares, en des proportions, exprimées en métaux, de 3 à 12 % en poids de cuivre, et pour ce qui est des autres chlorures présents, seuls ou en mélange, de 1 à 4 % en poids de métaux alcalins, de 0,05 à 2 % en poids de métaux alcalino-terreux et de 0,1 à 3 % en poids de métaux des terres rares, lesquels chlorures sont supportés sur des granules d'alumine, **caractérisés en ce que** la fraction de volume de pores constituée par les macropores, de plus de 50 et jusqu'à 10 000 nm de diamètre, des granules de catalyseur vaut de 20 à 40 % du volume total de pores, et **en ce que** le diamètre des pores, au maximum de la courbe de distribution de volume des macropores, vaut au moins 800 nm et jusqu'à 1500 nm, étant entendu que le volume de macropores est mesuré par porosimétrie au mercure.

2. Catalyseurs conformes à la revendication 1, qui présentent une masse volumique apparente de 0,65 à 0,8 g/mL.

3. Catalyseur conforme à la revendication 1 ou 2, dans lequel la proportion de cuivre vaut de 4 à 10 % en poids, et le métal ou les métaux alcalin(s) est ou sont du potassium et/ou du césium, le métal alcalino-terreux est du magnésium, et le métal des terres rares est du cérium.

4. Catalyseur conforme à l'une des revendications 1 à 3, dans lequel la proportion de potassium et/ou césium vaut de 0,5 à 3 % en poids, la proportion de magnésium vaut de 0,05 à 2 % en poids et la proportion de cérium vaut de 0,5 à 3 % en poids.

5. Catalyseur conforme à l'une des revendications 1 à 4, qui est un objet de forme creuse cylindrique de 4 à 6 mm de diamètre externe, 1 à 3 mm de diamètre interne et 4 à 7 mm de hauteur.

6. Catalyseur conforme à l'une des revendications 1 à 4, qui est un objet présentant une section transversale de cylindre trilobé, doté de lobes dont chacun est percé d'un trou traversant parallèle à l'axe du granule et équidistant des axes des autres lobes.

7. Utilisation de granules d'un catalyseur conforme à l'une des revendications 1 à 6 pour l'oxychloration d'éthylène en 1,2-dichloro-éthylène.
